# EUROPÄISCHE PATENTSCHRIFT

(11) **EP 1 485 710 B1**
(45) Veröffentlichungstag und Bekanntmachung des Hinweises auf die Patenterteilung: **11.04.2007**
(21) Anmeldenummer: 02708361.7
(22) Anmeldetag: 21.03.2002
(51) Int. Cl.: G01N 33/28, F01M 11/10

(54) **VORRICHTUNG UND VERFAHREN ZUR ERFASSUNG VON EIGENSCHAFTEN VON FLÜSSIGKEITEN**
DEVICE AND METHOD FOR DETECTING CHARACTERISTICS OF LIQUIDS
DISPOSITIF ET PROCEDE POUR DETERMINER DES PROPRIETES DE LIQUIDES

(43) Veröffentlichungstag der Anmeldung: 15.12.2004
(73) Patentinhaber: Fraunhofer-Gesellschaft zur Förderung der angewandten Forschung e.V., 80686 München (DE)
(72) Erfinder: KOPPI, Andreas, 80686 München (DE); MEIXNER, Leonhard, 81243 München (DE)
(74) Vertreter: Schoppe, Fritz
(86) Internationale Anmeldenummer: PCT/EP2002/003200
(87) Internationale Veröffentlichungsnummer: WO 2003/081232

(56) Entgegenhaltungen:
- WO-A-01/55718
- WO-A-96/28742
- DE-C- 19 704 461
- US-A- 3 686 926

## Beschreibung

Die vorliegende Erfindung betrifft eine Sensorvorrichtung und ein Verfahren zur Erfassung von Eigenschaften von Flüssigkeiten und insbesondere von Flüssigkeiten, die einem Alterungsprozeß unterliegenden, wobei der Alterungsprozeß durch die Erfassung von Eigenschaften der Flüssigkeiten erfaßt werden kann, wobei in den Flüssigkeiten elektrisch geladene Feststoffpartikel enthalten sein können. Beispiele derartiger Flüssigkeiten sind beispielsweise Schmieröle, besonders Schmieröle in Verbrennungsmotoren. Zur Erfassung des Alterungszustands von Flüssigkeiten, beispielsweise den oben genannten Schmierölen, ist aus dem Stand der Technik eine Anzahl unterschiedlicher Sensorsysteme bekannt, die verschiedene Parameter erfassen, um auf der Grundlage der erfaßten Parameter den Alterungszustand des Öls zu bestimmen.

Beispielsweise ist aus der DE 4131969 C2 eine Schmierölüberwachungseinrichtung bekannt, bei der die Parameter Druck, Temperatur und Viskosität sowie optional der pH-Wert, in situ im Motor erfaßt werden. Aus den erfaßten Parametern wird ein Ist-Zustand des Motoröls bestimmt, der mit einem Soll-Zustand verglichen wird, um basierend auf dem Vergleichsergebnis zu bestimmen, ob ein Ölwechsel erforderlich ist. Der Druck wird damit mittels einer Drucksensormembran gemessen, die Temperatur wird mittels Platinwiderständen gemessen, der pH-Wert wird mittels eines MOS-Transistors mit einer H⁺-sensitiven Gate-Elektrode gemessen, während die Viskosität aus der Dielektrizitätszahl, die mittels Kapazitätsstrukturen erfaßt wird, oder alternativ aus der Dämpfung von Schallwellen, die mittels Piezoschwingern erzeugt werden, gemessen wird.

Aus der WO 98/05953 ist ein Oberflächen-Flüssigkeitssensor bekannt, der einen Sendewandler und einen Empfangswandler aufweist, die durch jeweilige Interdigitalwandler gebildet sind. Ferner ist aus der WO 98/37412 eine Sensoreinrichtung zur Erfassung von Materialparametern eines flüssigen Mediums bekannt, bei der ein Oberflächenwellen-Flüssigkeitssensor zur Erfassung der Viskosität eines angrenzenden flüssigen Mediums verwendet wird, während einer der Interdigitalwandler des Oberflächenwellen-Flüssigkeitssensors ferner als kapazitiver Aufnehmer verwendet wird, um die Dielektrizitätszahl des anliegenden flüssigen Mediums zu bestimmen. Darüber hinaus lehrt die WO 98/37412, als weitere Parameter die Leitfähigkeit über die komplexe elektrische Impedanz des Interdigitalkondensators zu erfassen. Auch bei diesen Schriften wird basierend auf den erfaßten Parametern unter Berücksichtigung der Temperaturabhängigkeit ein Ist-Zustand mit einem Soll-Zustand verglichen, um einen Alterungszustand des flüssigen Mediums feststellen zu können.

In Motorenprüfstandsläufen wurde nun festgestellt, daß sich Schmutzpartikel an die Sensoroberfläche von SAW-Sensoren (SAW = surface acoustic wave = Oberflächenwelle) und IDK-Sensoren (IDK = Interdigitalkondensator), wie sie beispielsweise oben beschrieben wurden, anlagern und somit einen Belag bilden, der die Meßergebnisse der Sensoren bzw. des Sensorsystems verfälscht oder sogar den eigentlichen Meßeffekt ganz überlagern kann. Ein solches Verhalten wurde bei Messungen im Labor nicht festgestellt.

In der WO 01/55718 A1 sind nun Vorrichtungen und Verfahren zur Erfassung von durch Schmiermittel bedingten Belagbildungen auf Sensoroberflächen beschrieben. In dieser Schrift ist dargelegt, daß durch das Anlegen eines Potentials zwischen Sensor und Schmiermittel Schmutzpartikel zur Sensoroberfläche hin angezogen bzw. durch Umkehrung des Potentials abgestoßen werden können. Dabei wird davon ausgegangen, daß die Schmutzpartikel im flüssigen Medium, d. h. im Schmiermittel, immer gleiches Ladungsvorzeichen besitzen. Ist dies nicht der Fall, werden zwar bei einer Umkehrung der Polarität der Potentialdifferenz zwischen Sensorelement und Schmiermittel Schmutzpartikel von der Sensoroberfläche abgestoßen, aber andere Schmutzpartikel, welche die entgegengesetzte Polarität aufweisen, angezogen. Somit kommt es lediglich zu einem Austausch von Schmutzpartikeln, so daß die Sensoroberfläche nicht frei von Partikeln gehalten werden kann.

Ausgehend von dem oben genannten Stand der Technik liegt der vorliegenden Erfindung die Aufgabe zugrunde, eine Sensorvorrichtung und ein Verfahren zur Erfassung von Eigenschaften von Flüssigkeiten zu schaffen, die eine zuverlässige Bestimmung des Alterungszustands von Flüssigkeiten, wie z.B. von Schmierölen, ermöglichen.

Diese Aufgabe wird durch eine Sensorvorrichtung nach Anspruch 1 und ein Verfahren nach Anspruch 8 gelöst.

Die vorliegende Erfindung schafft eine Sensorvorrichtung zur Erfassung von Eigenschaften von Flüssigkeiten, mit folgenden Merkmalen:
einem Sensorelement zum Erfassen einer Eigenschaft einer mit einer Sensoroberfläche des Sensorelements in Berührung befindlichen Flüssigkeit; und
einem in der Flüssigkeit positionierbaren Elektrofilter, der mit einem solchen Potential beaufschlagbar ist, daß die Sensoroberfläche des Sensorelements vor geladenen Partikeln zumindest einer Polarität abgeschirmt ist.

Daneben schafft die vorliegende Erfindung ein Verfahren zum Erfassen von Eigenschaften von Flüssigkeiten, mit folgenden Schritten:
Anordnen einer Sensoroberfläche eines Sensorelements in Berührung mit einer Flüssigkeit zum Erfassen einer Eigenschaft der Flüssigkeit;
Positionieren eines Elektrofilters in der Flüssigkeit; und
Beaufschlagen des Elektrofilters mit einem solchen Potential, daß die Sensoroberfläche des Sensorelements vor geladenen Partikeln zumindest einer Polarität abgeschirmt ist.

Die vorliegende Erfindung geht auf die Erkenntnis zurück, daß in Flüssigkeiten, insbesondere in Schmiermitteln, enthaltene Schmutzpartikel unterschiedliche Ladungsvorzeichen besitzen können, so daß es durch das Anlegen eines Potentials zwischen Flüssigkeit und Sensorelement nicht zuverlässig möglich ist, die Sensoroberfläche des Sensorelements frei von Partikeln zu halten. Eine solche Freihaltung von Schmutzpartikeln ist jedoch notwendig, damit ein verwendeter Sensor die Eigenschaften der Flüssigkeit exakt und zuverlässig messen kann. Erfindungsgemäß wird die Sensoroberfläche vor geladenen Partikeln zumindest einer Polarität abgeschirmt, indem der Elektrofilter vorgesehen ist, der für die Partikel dieser Polarität eine stärkere elektrische Anziehungskraft besitzt als das Sensorelement bzw. die Sensoroberfläche desselben.

Die vorliegende Erfindung ermöglicht durch die Verwendung eines Elektrofilters, daß das Sensorelement von Schmutzpartikeln freigehalten wird, um eine störungsfreie Funktion zu gewährleisten und damit ein Meßergebnis zu erzielen, das nicht durch eine Betragsbildung verfälscht wird. Ferner ermöglicht die vorliegende Erfindung eine Aussage über den Partikelgehalt unter Verwendung des Prinzips, das in der WO 01/55718 A1 beschrieben ist, deren diesbezügliche Offenbarung hiermit durch Bezugnahme aufgenommen wird. Zusätzlich ist es jedoch gemäß der vorliegenden Erfindung möglich, gezielt Partikel einer Polarität von der Sensoroberfläche anziehen zu lassen, indem an Sensorelement und Elektrofilter entgegengesetzte Potentiale angelegt werden, sodaß Partikel einer Polarität zum Sensorelement angezogen werden, während Partikel der entgegengesetzten Polarität zum Elektrofilter angezogen werden. Somit kann eine zuverlässige Aussage über den Gehalt an Partikeln jeweils einer Polarität getroffen werden.

Alternativ ermöglicht die vorliegende Erfindung, geladene Partikel beider Polaritäten von der Sensoroberfläche fernzuhalten, so daß eine sichere Funktion des Sensors garantiert werden kann. Zu diesem Zweck umfaßt der erfindungsgemäße Elektrofilter vorzugsweise eine erste und eine zweite für die Flüssigkeit permeable Elektrodengitterstruktur. Die erste Elektrodengitterstruktur kann das Sensorelement zumindest teilweise umgebend angeordnet sein, während die zweite Elektrodengitterstruktur die erste Elektrodengitterstruktur zumindest teilweise umgebend angeordnet sein kann. An die beiden Elektrodengitterstrukturen werden dann Potentiale unterschiedlicher Polarität angelegt, so daß Partikel einer Polarität durch die eine Elektrodengitterstruktur angezogen werden, während Partikel der anderen Polarität durch die andere Elektrodengitterstruktur angezogen werden.

Im Gegensatz zur vorliegenden Erfindung ist es gemäß der WO 01/55718 A1 nur möglich, die Oberfläche von Sensoren von Partikeln freizuhalten, wenn in dem Schmiermittel nur Partikel einer Polarität vorhanden sind. Ist dies nicht der Fall, dann findet dort beim Umpolen des Potentials nur ein Austausch von Partikeln statt und es kann nie zu einer belagfreien Oberfläche kommen.

Die vorliegende Erfindung eignet sich speziell für den Einsatz im Zusammenhang mit SAW-Sensoren und IDK-Sensoren bzw. kombinierten SAW/IDK-Sensoren der oben genannten Art. Hinsichtlich des Aufbaus derartiger Sensoren sei beispielsweise auf die eingangs genannten Schriften verwiesen, deren diesbezügliche Offenbarung hiermit durch Bezugnahme aufgenommen wird. Insbesondere eignet sich die vorliegende Erfindung für den Einsatz solcher Sensoren bei der Ölzustandsbestimmung. Sie läßt sich jedoch auch auf andere Anwendungsgebiete bzw. Senderprinzipien, bei denen Sensoroberflächen vor einer Verschmutzung geschützt werden müssen, anwenden. Die vorliegende Erfindung ist für sämtliche Flüssigkeiten bzw. flüssigen Medien mit niedriger Leitfähigkeit einsetzbar. Die vorliegende Erfindung kann beispielsweise in der Labormeßtechnik und der Online-Überwachung von Ölen, Batterieflüssigkeiten und Bremsflüssigkeiten im Automobilbereich, Ölen in Hydraulikanlagen, in Getrieben und Lagern, sowie von Flüssigkeiten in der Verfahrenstechnik, der Farben- und Druck-Industrie, der Lebensmitteltechnologie, der Pharmazie und der Haushaltstechnik eingesetzt werden. Zweck der jeweiligen Überwachung unter Erfassung von Eigenschaften der Flüssigkeiten ist insbesondere die Ermittlung von bedarfsgerechten Wartungszyklen, eine Lebensdauererhöhung und eine Ressourcenschonung. Jedoch ist die vorliegende Erfindung auch zur Prozeßüberwachung, Kontrolle und Steuerung insbesondere in den oben genannten Anwendungsgebieten einsetzbar.

Bevorzugte Ausführungsbeispiele der vorliegenden Erfindung werden nachfolgend Bezug nehmend auf die beiliegende Zeichnung näher erläutert. Es zeigen:
- Fig. 1: eine schematische Darstellung eines bevorzugten Ausführungsbeispiels einer erfindungsgemäßen Sensoranordnung; und
- Fig. 2a): schematische Diagramme zur Veranschaulichung eines erfindugsgemäßen Verfahrens.

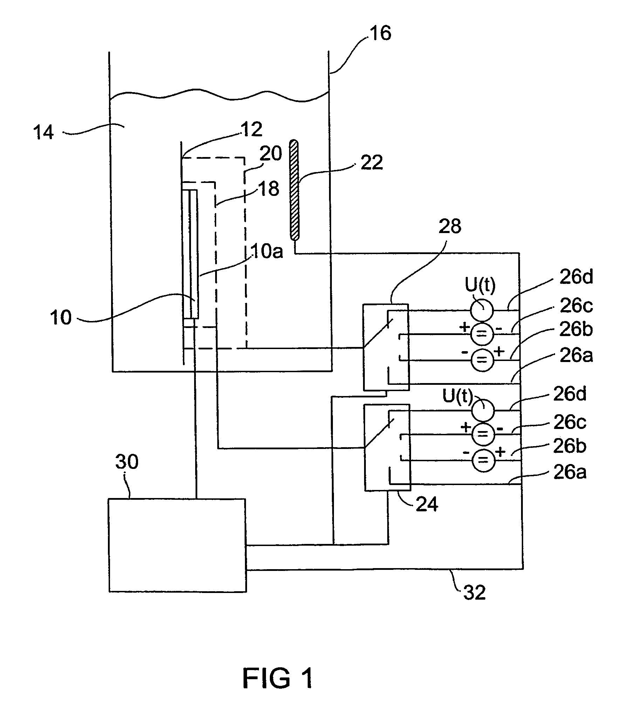

Wie in Fig. 1 gezeigt ist, ist ein Sensorelement 10, das an einem Träger 12 angebracht ist, in einem Flüssigkeitsbehältnis 16 derart angeordnet, daß eine Sensoroberfläche 10a in Berührung mit einer in dem Flüssigkeitsbehältnis befindlichen flüssigen Medium 14 ist. Soll die erfindungsgemäße Sensoranordnung zur Online-Überwachung des Motoröls eines Kraftfahrzeugs verwendet werden, kann das Flüssigkeitsbehältnis 16 beispielsweise durch die Ölwanne des Kraftfahrzeugs gebildet sein. Das Sensorelement 10 kann ein übliches Sensorelement zum Erfassen von physikalischen Parametern eines Schmiermittels sein, beispielsweise ein Oberflächenwellensensor, wie er in den Druckschriften WO 98/05953 und WO 98/37412 beschrieben ist. Der Sensor kann jedoch auch eine Kombination aus Oberflächenwellensensor und kapazitivem Sensor sein, wie er beispielsweise in der WO 01/55718 beschrieben ist. Jedoch kann das Sensorelement 10 ein beliebiger Sensor zur Erfassung physikalischer Parameter sein, dessen Ausgangssignal von einer an demselben, bzw. einer Sensoroberfläche desselben, anliegenden Flüssigkeit abhängt, d. h. der auf Änderungen der physikalischen Randbedingungen an der Grenzfläche zwischen Sensor und Flüssigkeit reagiert. Beispielsweise kommt diesbezüglich auch ein Schwingquarz als Sensorelement zur Erfassung der Viskosität in Betracht.

Wie ferner in Fig. 1 gezeigt ist, sind in Berührung mit der Flüssigkeit 14 ferner ein erstes Gitter 18 und ein zweites Gitter 20 angeordnet. Die Gitter 18 und 20 weisen eine solche Struktur auf, daß sie für die Flüssigkeit 14 permeabel sind, so daß die Flüssigkeit 14 zu der freiliegenden Oberfläche des Sensorelements 10 gelangen kann. Ferner können die Gitter 18 und 20 als Elektrodengitterstrukturen bezeichnet werden, da dieselben ein leitfähiges Material aufweisen, um sie mit einem Potential beaufschlagen zu können.

Bei dem gezeigten Ausführungsbeispiel sind die Gitter 18 und 20 ebenfalls an dem Träger 12 angebracht, wobei das erste Gitter 18 benachbart zu dem Sensorelement 10 und das zweite Gitter 20 außerhalb des ersten Gitters 20 angeordnet ist, so daß das erste Gitter zwischen dem zweiten Gitter und dem Sensorelement angeordnet ist. Das Gitter 18 ist vorzugsweise der gesamten Sensoroberfläche des Sensorelements 10 gegenüberliegend angeordnet, während das Gitter 20 über der gesamten Fläche 18, die der Sensoroberfläche des Sensorelements 10 gegenüberliegt, angeordnet ist. Beispielsweise können die Gitterstrukturen 18 und 20 als das Sensorelement 10 zusammen mit dem Träger 12 umgebende Käfigstrukturen ausgebildet sein, so daß das die Flüssigkeit nur über die Gitterstrukturen zu der Sensoroberfläche gelangen kann. Ferner ist ein Flüssigkeitskontakt 22 vorgesehen, so daß eine Potentialdifferenz zwischen Flüssigkeit und erster Gitterstruktur, zweiter Gitterstruktur und/oder Sensorelement anlegbar ist.

Eine erste Schalteinrichtung 24 ist mit dem ersten Gitter 18 verbunden. Über die Schalteinrichtung 24 ist ein Kurzschluß 26a, ein Potential 26b einer ersten Polarität, ein Potential 26c einer zweiten zu der ersten Polarität unterschiedlichen bzw. entgegengesetzten Polarität und ein zeitlich variierendes Potential 26d zwischen das erste Gitter 18 und den Flüssigkeitskontakt 22 schaltbar. In vergleichbarer Weise ist eine zweite Schalteinrichtung 28 vorgesehen, über die ebenfalls unterschiedliche Potentiale 26a bis 26d zwischen das zweite Gitter 20 und den Flüssigkeitskontakt 22 schaltbar sind. Durch die Schalteinrichtungen 24 und 28 können unabhängig voneinander unterschiedliche Potentialdifferenzen zwischen dem ersten Gitter 18 und dem Flüssigkeitskontakt 22 sowie zwischen dem zweiten Gitter 20 und dem Flüssigkeitskontakt 22 angelegt werden.

Ferner weist das in Fig. 1 gezeigte Ausführungsbeispiel einer Sensoranordnung eine Sensorsteuerung 30 auf, die mit der ersten und der zweiten Schalteinrichtung 24 und 28 verbunden ist, um ein jeweils erforderliches Potential zwischen dem jeweiligen Gitter und dem Flüssigkeitskontakt 22 anzulegen. Die Sensorsteuerung 30 ist ferner mit dem Sensorelement 10 verbunden, um Sensorsignale von dem Sensorelement 10 aufzunehmen. Ferner kann die Sensorsteuerung ausgelegt sein, um eine vorbestimmte Potentialdifferenz zwischen dem Sensorelement 10 und dem Flüssigkeitskontakt 22 zu bewirken, was durch eine Verbindungsleitung 32 angezeigt ist.

Im folgenden wird auf den Betrieb einer erfindungsgemäßen Sensoranordnung näher eingegangen. Wie bereits eingangs erwähnt wurde, wird davon ausgegangen, daß die in der Flüssigkeit 14, bei der es sich beispielsweise um Schmieröl handelt, befindlichen Schmutzpartikel eine Ladung besitzen, wobei diese Ladung positiv und/oder negativ sein kann. Durch einen Elektrofilter, der erfindungsgemäß durch die beiden Gitter 18 und 20 realisiert ist, können sämtliche geladenen Schmutzpartikel von der Sensoroberfläche des Sensorelements 10 abgehalten werden. Zu diesem Zweck wird das Potential des Sensors neutral geschaltet, d. h. auf gleiches Potential wie die Flüssigkeit. Zwischen dem Gitter 18 und der Flüssigkeit 14 wird beispielsweise eine negative Potentialdifferenz angelegt, indem durch die Schalteinrichtung 24 die Potentialquelle 26b zwischen das erste Gitter 18 und den Flüssigkeitskontakt 22 geschaltet wird. Somit werden positiv geladene Partikel zu dem ersten Gitter 18 hin angezogen. Dagegen wird zwischen dem zweiten Gitter 20 und dem Flüssigkeitskontakt 22 eine positive Potentialdifferenz angelegt, indem die erste Schalteinrichtung 28 die Potentialquelle 26c zwischen das zweite Gitter 20 und den Flüssigkeitskontakt 22 schaltet. Dadurch werden negativ geladene Partikel zu dem zweiten Gitter 20 hin angezogen. Beide Gitter zusammen schirmen somit das Sensorelement 10 von geladenen Partikeln beider Polaritäten in der Flüssigkeit ab, so daß der Sensor ohne den Einfluß eines Belags die Eigenschaften der Flüssigkeit messen kann. Neben der Wirkung, daß jeweils Partikel einer Polarität durch jedes der beiden Gitter angezogen werden, besitzen die beiden Gitter ferner die Wirkung, daß Partikel, die die gleiche Polarität wie das jeweilige Gitter besitzen, nach außen abgestoßen werden.

Bei dem dargestellten Ausführungsbeispiel kann die Flüssigkeit ohne weiteres auf die Sensoroberfläche gelangen, da der Elektrofilter durch zwei für die Flüssigkeit permeable Gitterstrukturen gebildet ist. Alternativ könnte der Elektrofilter durch beliebige Elektrodenstrukturen gebildet sein, die in einer entsprechenden Beziehung bezüglich des Sensorelements 10 angeordnet sind, so daß zum einen sichergestellt ist, daß das Sensorelement gegen Schmutzpartikel abgeschirmt werden kann, und zum anderen sichergestellt ist, daß die zu messende Flüssigkeit noch zu der Oberfläche gelangen kann. Beispielsweise könnten zu diesem Zweck Elektrodenstäbe oder Elektrodenplatten, die einen Durchfluß zwischen Sensoroberfläche und Elektrodenplatte ermöglichen, vorgesehen sein. Wiederum alternativ könnte der Elektrofilter durch zwei unabhängig voneinander mit einem Potential beaufschlagbare Elektrodenstrukturen gebildet sein, die in einer einzigen Ebene bezüglich der Sensoroberfläche des Sensorelements 10 sozusagen verschachtelt miteinander angeordnet sind.

Der Flüssigkeitskontakt 22 kann als zusätzliche Elektrode in das Flüssigkeitsbehältnis 16 eingebracht sein. Ferner kann der Flüssigkeitskontakt auch durch das Flüssigkeitsbehältnis selbst gebildet sein, falls dieses aus einem leitfähigen Material besteht. Der Flüssigkeitskontakt ist vorgesehen, um zu ermöglichen, daß eine jeweilige Potentialdifferenz zwischen Sensorelement, erstem Gitter und/oder zweitem Gitter angelegt werden kann, wobei die exakte Ausgestaltung und Anordnung desselben nicht erheblich ist.

Alternativ zu der in Fig. 1 dargestellten Ausführungsform mit zwei Elektrodenstrukturen, von denen jede geladene Partikel einer Polarität anzieht, kann ein Elektrodenfilter durch nur eine Elektrodenstruktur realisiert sein, die mit einem Potential beaufschlagt wird, so daß Partikel einer ersten Polarität angezogen werden, während Partikel einer zweiten, der ersten Polarität entgegengesetzten Polarität abgestoßen werden. In einem solchen Fall kann das Sensorelement mit einem geringeren Potential gleicher Polarität wie der Filter beaufschlagt werden, so daß Partikel der zweiten Polarität abgestoßen werden, Partikel der ersten Polarität jedoch nicht von dem Sensorelement angezogen werden, da die Anziehungswirkung des Filters nicht übertroffen wird. Somit kann auch in einem solchen Fall zuverlässig sichergestellt werden, daß weder Partikel der einen Polarität noch der anderen Polarität zu der Sensoroberfläche gelangen.

Durch das Anlegen einer geeigneten Abfolge von Potentialen an den Elektrofilter, d. h. die Gitter 18 und 20, können auch Partikel aus dem Raum, den die beiden hintereinander liegenden Filter umschließen, d. h. dem Innenraum, nach außen "gezogen" werden. Dies kann beispielsweise folgendermaßen erfolgen: die innere Gitterelektrodenstruktur 18 wird neutral geschaltet, während an die äußere Gitterelektrodenstruktur 20 ein positives Potential angelegt wird. Alle negativ geladenen Partikel aus dem Raum, den die zweite Gitterelektrodenstruktur 20 umschließt, werden von dieser Gitterelektrodenstruktur angezogen. Werden dann beide Gitter 18 und 20 gegenüber der Flüssigkeit 14 auf ein negatives Potential gelegt, dann werden die Partikel nach außen abgestoßen. Um entgegengesetzt geladen Partikel aus dem Innenraum zu entfernen, wird entsprechend mit Potentialen umgekehrten Vorzeichens gearbeitet. Ein solcher Abzug der Partikel aus dem Innenraum kann beispielsweise beim Einschalten des Systems wichtig sein. Ferner können die einzelnen Elektrodenstrukturen des Filters auf diese Weise auch gereinigt, d. h. von angelagerten Partikeln befreit werden.

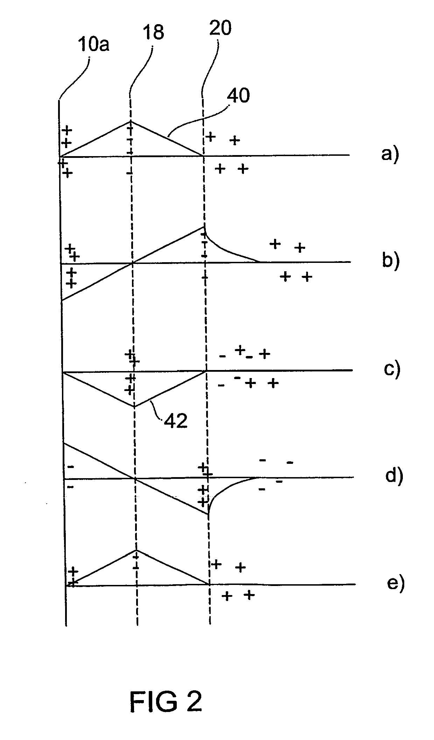

Ein Ausführungsbeispiel der vorliegenden Erfindung, bei dem das Elektrofilter als Pumpe verwendet ist, ist in den Fig. 2a) bis 2e) gezeigt. Dabei funktioniert das Elektrofilter als Pumpe analog zu einem CCD-Element (CCD = charge coupled device).

Bei dem in Fig. 2 gezeigten erfindungsgemäßen Pumpverfahren werden unterschiedliche Potentiale an die Sensoroberfläche 10a, das erste Elektrodengitter 18 und das zweite Elektrodengitter 20 angelegt, um Partikel beider Polaritäten aus dem Raum zwischen der Sensoroberfläche 10a und dem zweiten Elektrodengitter 20 zu pumpen. Bei dem gezeigten Ausführungsbeispiel wird ferner das Potential der Flüssigkeit außerhalb des zweiten Elektrodengitters 20, d.h. in den Figuren rechts davon, auf Null gehalten, was beispielsweise durch einen großflächigen Flüssigkeitskontakt (in Fig. 2 nicht gezeigt) erfolgen kann.

In einem ersten Schritt, der in Fig. 2a) gezeigt ist, wird zunächst ein positives Potential an das erste Elektrodengitter 18 angelegt, während an die Sensoroberfläche 10a und das zweite Elektrodengitter 20 kein Potential angelegt wird. Der sich ergebende Potentialverlauf ist mit dem Bezugszeichen 40 bezeichnet. Dadurch werden negativ geladene Partikel durch das Elektrodengitter 18 angezogen, während positiv geladene Partikel durch dasselbe abgestoßen werden.

In einem nachfolgenden Schritt, der in Fig. 2b) gezeigt ist, wird ein negatives Potential an die Sensoroberfläche 10a angelegt, ein positives Potential an das zweite Elektrodengitter 20 angelegt, während an das erste Elektrodengitter 18 kein Potential angelegt wird. Dadurch werden die negativ geladenen Partikel von dem ersten Elektrodengitter 18 zu dem zweiten Elektrodengitter 20 verschoben.

Nachfolgend wird ein negatives Potential an das erste Elektrodengitter 18 angelegt, während an die Sensoroberfläche 10a und das zweite Elektrodengitter 20 kein Potential angelegt wird. Der sich dabei ergebende Potentialverlauf 42 ist in Fig. 2c) dargestellt. Dabei werden die positiv geladenen Partikel auf der Sensoroberfläche 10a zu dem ersten Elektrodengitter 18 verschoben.

In einem anschließenden Schritt, der in Fig. 2d) gezeigt ist, wird ein positives Potential an die Sensoroberfläche 10a angelegt, ein negatives Potential wird an das zweite Elektrodengitter 20 angelegt, während an das erste Elektrodengitter 18 kein Potential angelegt wird. Dadurch werden die positiv geladenen Partikel von dem ersten Elektrodengitter 18 zu dem zweiten Elektrodengitter 20 verschoben, da sie von demselben angezogen werden, während sie von der Sensoroberfläche 10a abgestoßen werden.

In einem anschließenden Schritt, der in Fig. 2e) gezeigt ist, kann das Verfahren wieder von vorne beginnen, indem Potentiale, die denen in Fig. 2a) entsprechen, angelegt werden.

Zusammenfassend ist festzustellen, daß bei dem erfindungsgemäßen Pumpverfahren die Potentialverläufe 40 und 42 von links nach rechts zwischen Sensoroberfläche und zweitem Elektrodengitter verschoben werden, um die beschriebene Pumpwirkung zu erreichen. Die angelegten Potentiale sind dabei so zu wählen, daß zunächst Partikel der ersten Polarität von der Sensoroberfläche über das erste Elektrodengitter zum zweiten Elektrodengitter und nach außen verschoben werden, und nachfolgend Partikel der zweiten Polarität von der Sensoroberfläche über das erste Elektrodengitter zum zweiten Elektrodengitter und nach außen verschoben werden. Zu diesem Zweck werden jeweils Potentiale unterschiedlicher Polarität und Höhe angelegt, so daß zwischen zwei benachbarten Elementen die erforderlic e "Nettoverschiebung" erreicht wird. Die oben beschriebenen angelegten Potentiale sind dabei lediglich beispielhafter Natur. Um eine entsprechende von Partikeln einer Polarität von einem ersten Element zu einem zweiten Element zu erreichen, muß lediglich dafür gesorgt werden, daß zwischen den beiden Elementen eine geeignete Potentialdifferenz herrscht.

Falls es nicht möglich ist, das Potential des Sensors selbst zu takten, d.h. ein Potential an die Sensoroberfläche 10a anzulegen, so kann dies auch durch ein weiteres Elektrodengitter erfolgen, das zwischen der Sensoroberfläche und dem ersten Elektrodengitter angeordnet ist.

Der Kontakt zum Beaufschlagen der Flüssigkeit mit einem Potential kann ebenfalls als Elektrodengitter ausgebildet sein. Somit sind zwei bis vier Gitter notwendig, um mit dem erfindungsgemäßen Elektrofilter eine Pumpwirkung zu erzielen, wobei mit dem erfindungsgemäßen Elektrofilter eine Pumpwirkung zu erzielen. Je mehr Gitter vorgesehen und mit den entsprechenden Potentialen beaufschlagt werden, d.h. getaktet werden, desto effektiver ist die Pumpfwirkung.

Das Umschalten zwischen den unterschiedlichen Potentialen kann getaktet erfolgen. Alternativ kann eine kontinuierliche Potentialänderung bewirkt werden, um den erforderlichen Potentialverlauf stetig zu durchfahren. Die Taktzeit ist derart zu wählen, daß die Driftzeit der Partikel im elektrischen Feld von einem Gitter zum nächsten etwa der Zeit von einem Takt zum nächsten entspricht oder größer ist. Bei einem stetigen Potentialverlauf gilt das entsprechende.

Neben den oben beschriebenen Ausführungsbeispielen, bei denen die Oberfläche des Sensorelements jeweils vollstän ig frei von geladenen Partikeln gehalten wurde, ist die vorliegende Erfindung auch einsetzbar, um analog der in der WO 01/55718 A1 beschriebenen Technik die Belagbildung auf der Sensoroberfläche des Sensorelements 10 zu untersuchen. Somit kann zusätzlich eine Aussage über den Partikelgehalt getroffen werden.

## Patentansprüche

1. Sensorvorrichtung zur Erfassung von Eigenschaften von Flüssigkeiten (14), mit folgenden Merkmalen:
einem Sensorelement (10) zum Erfassen einer Eigenschaft einer mit einer Sensoroberfläche (10a) des Sensorelements (10) in Berührung befindlichen Flüssigkeit (14) ;
einem von dem Sensorelement (10) getrennten in der Flüssigkeit positionierbaren Elektrofilter (18, 20); und
einer Einrichtung zum Beaufschlagen des Elektrofilters mit einem solchen Potential, dass die Sensoroberfläche (10a) des Sensorelements (10) vor geladenen Partikeln zumindest einer Polarität abgeschirmt ist.

2. Sensorvorrichtung nach Anspruch 1, bei dem der Elektrofilter eine erste Elektrodenstruktur (18) aufweist.

3. Sensorvorrichtung nach Anspruch 2, bei der der Elektrofilter eine zweite Elektrodenstruktur (20) aufweist, wobei die erste und zweite Elektrodenstruktur (18, 20) mit unterschiedlichen Potentialen beaufschlagbar sind.

4. Sensorvorrichtung nach Anspruch 3, die eine Einrichtung (24, 28, 30) zum Anlegen von Potentialen unterschiedlicher Polarität an die erste und die zweite Elektrodenstruktur aufweist.

5. Sensorvorrichtung nach Anspruch 4, bei der die Einrichtung zum Anlegen von Potentialen eine Steuereinrichtung (30) und Schalter (24, 28) aufweist, wobei die Steuereinrichtung (30) konfiguriert ist, um die Schalter zu steuern, um eine solche Abfolge von Potentialen an die erste und die zweite Elektrodenstruktur (18, 20) anzulegen, daß geladene Partikel einer oder beider Polaritäten aus dem Raum zwischen der ersten Elektrodengitterstruktur (18) und dem Sensorelement (10) gezogen werden.

6. Sensorvorrichtung nach einem der Ansprüche 2 bis 5, die ferner eine Einrichtung zum Anlegen von Potentialen unterschiedlicher Polarität an die erste Elektrodengitterstruktur (18) und das Sensorelement (10) aufweist.

7. Sensorvorrichtung nach einem der Ansprüche 2 bis 6, bei der die Elektrodenstrukturen (18, 20) für die Flüssigkeit permeable Elektrodengitterstrukturen aufweisen.

8. Verfahren zum Erfassen von Eigenschaften von Flüssigkeiten, mit folgenden Schritten:
Anordnen einer Sensoroberfläche (10a) eines Sensorelements (10) in Berührung mit einer Flüssigkeit (14) zum Erfassen einer Eigenschaft der Flüssigkeit (14);
Positionieren eines Elektrofilters (18, 20) in der Flüssigkeit (14) getrennt von dem Sensorelement (10); und
Beaufschlagen des Elektrofilters (18, 20) mit einem solchen Potential, daß die Sensoroberfläche (10a) des Sensorelements (10) vor geladenen Partikeln zumindest einer Polarität abgeschirmt ist.

9. Verfahren nach Anspruch 8, bei dem der Elektrofilter eine erste Elektrodenstruktur und eine zweite Elektrodenstruktur aufweist, wobei das Verfahren ferner den Schritt des Anlegens von unterschiedlichen Potentialen an die beiden Elektrodenstrukturen aufweist.

10. Verfahren nach Anspruch 9, das vor dem Schritt des Anlegens von Potentialen unterschiedlicher Polarität an beide Elektrodenstrukturen einen Schritt des Anlegens einer Abfolge von Potentialen an die erste und die zweite Elektrodenstruktur (18, 20) mit folgenden Teilschritten aufweist:
a) Anlegen eines Potentials erster Polarität an die zweite Elektrodenstruktur und eines Null-Potentials an die erste Elektrodenstruktur (18); und
b) Anlegen eines Potentials einer zweiten, zu der ersten unterschiedlichen Polarität an die erste und die zweite Elektrodenstruktur (18, 20).

11. Verfahren nach Anspruch 10, das ferner den Schritt des Wiederholens der Schritte a) und b) mit Potentialen umgekehrter Polarität aufweist.

12. Verfahren nach Anspruch 8, bei dem das Elektrofilter eine erste Elektrodenstruktur (18), die zwischen Sensoroberfläche (10a) und einer zweiten Elektrodenstruktur (20) angeordnet ist, aufweist, wobei das Verfahren Schritte des Anlegens von Potentialen an die erste und die zweite Elektrodenstruktur aufweist, um geladene Partikel beider Polaritäten aus einem Raum zwischen der zweiten Elektrodenstruktur (20) und der Sensoroberfläche (10a) zu pumpen.

13. Verfahren nach Anspruch 12, das folgende Schritte aufweist:
Anlegen eines Potentials einer ersten Polarität an die erste Elektrodenstruktur (18);
Anlegen eines Potentials der ersten Polarität an die zweite Elektrodenstruktur (20) und Anlegen eines Potentials einer zweiten von der ersten verschiedenen Polarität an die Sensoroberfläche (10a);
Anlegen eines Potentials der zweiten Polarität an die erste Elektrodenstruktur (18); und
Anlegen eines Potentials der ersten Polarität and die Sensoroberfläche (10a) und eines Potentials der zweiten Polarität an die zweite Elektrodenstruktur (20).

14. Verfahren nach Anspruch 12, bei dem das Elektrofilter ferner eine dritte Elektrodenstruktur zwischen der Sensoroberfläche und der ersten Elektrodenstruktur aufweist, wobei das Verfahren folgende Schritte aufweist:
Anlegen eines Potentials einer ersten Polarität an die erste Elektrodenstruktur (18);
Anlegen eines Potentials der ersten Polarität an die zweite Elektrodenstruktur (20) und Anlegen eines Potentials einer zweiten von der ersten verschiedenen Polarität an die dritte Elektrodenstruktur;
Anlegen eines Potentials der zweiten Polarität an die erste Elektrodenstruktur (18); und
Anlegen eines Potentials der ersten Polarität and die dritte Elektrodenstruktur und eines Potentials der zweiten Polarität an die zweite Elektrodenstruktur (20).

## Claims

1. A sensor device for detecting characteristics of liquids (14), comprising:
a sensor element (10) for detecting a characteristic of a liquid (14) being in contact with a sensor surface (10a) of the sensor element (10);
an electrostatic filter (18, 20) separate from the sensor element (10), which can be positioned in the liquid; and
a means for providing the electrostatic filter with such a potential that the sensor surface (10a) of the sensor element (10) is shielded from charged particles of at least one polarity.

2. The sensor device according to claim 1, wherein the electrostatic filter comprises a first electrode structure (18).

3. The sensor device according to claim 2, wherein the electrostatic filter comprises a second electrode structure (20), wherein the first and second electrode structures (18, 20) can be provided with different potentials.

4. The sensor device according to claim 3, comprising a means (24, 28, 30) for applying potentials of different polarities to the first and second electrode structures.

5. The sensor device according to claim 4, wherein the means for applying potentials comprises a control means (30) and switches (24, 28), wherein the control means (30) is configured to control the switches in order to apply such a sequence of potentials to the first and second electrode structures (18, 20) that charged particles of one or both polarities are drawn from the space between the first electrode grid structure (18) and the sensor element (10).

6. The sensor device according to one of claims 2 to 5, further comprising a means for applying potentials of different polarities to the first electrode grid structure (18) and the sensor element (10).

7. The sensor device according to one of claims 2 to 6, wherein the electrode structures (18, 20) comprise electrode grid structures permeable for the liquid.

8. A method for detecting characteristics of liquids, comprising:
arranging a sensor surface (10a) of a sensor element (10) in contact with a liquid (14) for detecting a characteristic of the liquid (14);
positioning an electrostatic filter (18, 20) in the liquid (14) separate from the sensor element (10); and
providing the electrostatic filter (18, 20) with such a potential that the sensor surface (10a) of the sensor element (10) is shielded from charged particles of at least one polarity.

9. The method according to claim 8, wherein the electrostatic filter comprises a first electrode structure and a second electrode structure, wherein the method further comprises the step of applying different potentials to the two electrode structures.

10. The method according to claim 1, which, prior to the step of applying potentials of different polarities to both electrode structures, comprises a step of applying a sequence of potentials to the first and second electrode structures (18, 20), comprising the following sub-steps:
a) applying a potential of a first polarity to the second electrode structure and a zero potential to the first electrode structure (18); and
b) applying a potential of a second polarity different from the first to the first and second electrode structures (18, 20).

11. The method according to claim 10, further comprising the step of repeating steps a) and b) with potentials of reverse polarities.

12. The method according to claim 8, wherein the electrostatic filter comprises a first electrode structure (18) arranged between a sensor surface (10a) and a second electrode structure (20), wherein the method comprises steps of applying potentials to the first and second electrode structures in order to pump charged particles of both polarities from a space between the second electrode structure (20) and the sensor surface (10a).

13. The method according to claim 12, comprising:
applying a potential of a first polarity to the first electrode structure (18);
applying a potential of the first polarity to the second electrode structure (20) and applying a potential of a second polarity different from the first to the sensor surface (10a);
applying a potential of the second polarity to the first electrode structure (18); and
applying a potential of the first polarity to the sensor surface (10a) and a potential of the second polarity to the second electrode structure (20).

14. The method according to claim 12, wherein the electrostatic filter further comprises a third electrode structure between the sensor surface and the first electrode structure, wherein the method comprises:
applying a potential of a first polarity to the first electrode structure (18);
applying a potential of the first polarity to the second electrode structure (20) and applying a potential of a second polarity different from the first to the third electrode structure;
applying a potential of the second polarity to the first electrode structure (18); and
applying a potential of the first polarity to the third electrode structure and a potential of the second polarity to the second electrode structure (20).

## Revendications

1. Dispositif détecteur pour déterminer des propriétés de liquides (14), aux caractéristiques suivantes :
un élément détecteur (10) destiné à déterminer une propriété d'un liquide (14) se trouvant en contact avec une surface de détection (10a) de l'élément détecteur (10) ;
un filtre électrostatique (18 ; 20) pouvant être positionné dans le liquide, séparé de l'élément détecteur (10) ; et
un dispositif destiné à soumettre le filtre électrostatique à un potentiel tel que la surface de détection (10a) de l'élément détecteur (10) soit protégée contre les particules chargées d'au moins une polarité.

2. Dispositif détecteur selon la revendication 1, dans lequel le filtre électrostatique présente une première structure d'électrode (18).

3. Dispositif détecteur selon la revendication 2, dans lequel le filtre électrostatique présente une deuxième structure d'électrode (20), la première et la deuxième structure d'électrode (18, 20) pouvant être soumises à des potentiels différents.

4. Dispositif détecteur selon la revendication 3, présentant un dispositif (24, 28, 30) destiné à appliquer des potentiels de polarité différente à la première et la deuxième structure d'électrode.

5. Dispositif détecteur selon la revendication 4, dans lequel le dispositif destiné à appliquer des potentiels présente un dispositif de commande (30) et un commutateur (24, 28), le dispositif de commande (30) étant configuré pour commander les commutateurs de manière à appliquer à la première et à la deuxième structure d'électrode (18, 20) une succession de potentiels telle que les particules chargées d'une ou des deux polarités soient extraites hors de l'espace entre la première structure de grille d'électrode (18) et l'élément détecteur (10).

6. Dispositif détecteur selon l'une des revendications 2 à 5, présentant, par ailleurs, un dispositif destiné à appliquer des potentiels de polarité différente à la première structure de grille d'électrode (18) et l'élément détecteur (10).

7. Dispositif détecteur selon l'une des revendications 2 à 6, dans lequel les structures d'électrode (18, 20) présentent des structures de grille d'électrode perméables au liquide.

8. Procédé pour détecter des propriétés de liquides, aux étapes suivantes consistant à :
disposer une surface de détection (10a) d'un élément détecteur (10) en contact avec un liquide (14), pour détecter une propriété du liquide (14) ;
positionner un filtre électrostatique (18, 20) dans le liquide, séparé de l'élément détecteur (10); et
soumettre le filtre électrostatique (18, 20) à un potentiel tel que la surface de détection (10a) de l'élément détecteur (10) soit protégée contre les particules chargées d'au moins une polarité.

9. Procédé selon la revendication 8, dans lequel le filtre électrostatique présente une première structure d'électrode et une deuxième structure d'électrode, le procédé présentant, par ailleurs, l'étape consistant à appliquer des potentiels différents aux deux structures d'électrode.

10. Procédé selon la revendication 9, présentant, avant l'étape consistant à appliquer des potentiels de polarité différente aux deux structures d'électrode, une étape consistant à appliquer une succession de potentiels à la première et la deuxième structure d'électrode (18, 20), aux étapes partielles suivantes consistant à :
a) appliquer un potentiel de la première polarité à la deuxième structure d'électrode et un potentiel nul sur la première structure d'électrode (18); et
b) appliquer un potentiel d'une deuxième polarité, différente de la première polarité, à la première et la deuxième structure d'électrode (18, 20).

11. Procédé selon la revendication 10, présentant, par ailleurs, l'étape consistant à répéter les étapes a) et b) avec des potentiels de polarité inversée.

12. Procédé selon la revendication 8, dans lequel le filtre électrostatique présente une première structure d'électrode (18) qui est disposée entre la surface de détection (10a) et une deuxième structure d'électrode (20), le procédé présentant des étapes consistant à appliquer des potentiels à la première et la deuxième structure d'électrode, pour pomper des particules chargées des deux polarités hors d'un espace entre la deuxième structure d'électrode (20) et la surface de détection (10a).

13. Procédé selon la revendication 12, présentant les étapes suivantes consistant à :
appliquer un potentiel d'une première polarité à la première structure d'électrode (18) ;
appliquer un potentiel de la première polarité à la deuxième structure d'électrode (20) et appliquer un potentiel d'une deuxième polarité, différente de la première polarité, à la surface de détection (10a);
appliquer un potentiel de la deuxième polarité à la première structure d'électrode (18) ; et
appliquer un potentiel de la première polarité à la surface de détection (10a) et un potentiel de la deuxième polarité à la deuxième structure d'électrode (20).

14. Procédé selon la revendication 12, dans lequel le filtre électrostatique présente, par ailleurs, une troisième structure d'électrode entre la surface de détection et la première structure d'électrode, le procédé présentant les étapes suivantes consistant :
appliquer un potentiel d'une première polarité à la première structure d'électrode (18) ;
appliquer un potentiel de la première polarité à la deuxième structure d'électrode (20) et appliquer un potentiel d'une deuxième polarité, différente de la première polarité, à la troisième structure d'électrode ;
appliquer un potentiel de la deuxième polarité à la première structure d'électrode (18) ; et
appliquer un potentiel de la première polarité à la troisième structure d'électrode et un potentiel de la deuxième polarité à la deuxième structure d'électrode (20).
